Europäisches Patentamt

European Patent Office

Office européen des brevets

(11) Publication number: **0 273 679**
**A2**

## (12) EUROPEAN PATENT APPLICATION

(21) Application number: **87311290.8**

(22) Date of filing: **22.12.87**

(51) Int. Cl.⁴: **C12M 1/12 , C12M 1/40**

(30) Priority: **29.12.86 US 947249**

(43) Date of publication of application:
**06.07.88 Bulletin 88/27**

(84) Designated Contracting States:
**CH DE FR GB LI**

(71) Applicant: **EASTMAN KODAK COMPANY**
**Patent Department 343 State Street**
**Rochester New York 14650(US)**

(72) Inventor: **Chen, Stephen Po-Kwok c/o**
**EASTMAN KODAK COMPANY**
**Patent Department 343 State Street**
**Rochester New York 14650(US)**

(74) Representative: **Davis, Ian Ellison et al**
**Kodak Limited Patent Department Headstone**
**Drive**
**Harrow Middlesex HA1 4TY(GB)**

(54) **Improved method for effecting enzymatic reactions and an enzymatic reactor useful therein.**

(57) A method for effecting enzymatic reactions can be carried out using a microporous enzymatic reactor which comprises a porous support having thereon a crosslinked polymeric membrane containing solubilizing groups and having an enzyme attached thereto. In a preferred embodiment, the membrane is composed of polymers prepared from acrylamide or methacrylamide monomers containing active methylene groups, and solubilizing ethylenically unsaturated monomers. The method is carried out by causing a feed stream containing a substrate for the enzyme in a first solvent to flow rapidly and continuously over the surface of the membrane. A product of the enzymatic reaction occurring in the membrane is collected in a product stream containing a second solvent which is immiscible with the first solvent, the product stream being separated from the feed stream by the enzymatic membrane.

# IMPROVED METHOD FOR EFFECTING ENZYMATIC REACTIONS AND AN ENZYMATIC REACTOR USEFUL THEREIN

This invention relates to a method for carrying out chemical reactions using, as catalysts, enzymes that are immobilized in a membrane. The method is particularly useful for separating racemic mixtures of enantiomorphs. This invention also relates to a microporous enzymatic reactor useful in the method, and to a method for preparing the reactor.

In recent years, the art of attaching enzymes to insoluble supporting materials, such as membranes, has advanced sufficiently to provide a number of commercial processes. The primary advantage of such processes is that the enzyme used to catalyze desired reactions can be retained indefinitely in the reaction vessel while being contacted by a continuously flowing stream of substrate.

A wide variety of materials have been used as the supporting materials to which the enzyme is attached. For example, porous inorganic materials and synthetic and natural polymers have been used. Workers in the art have sought polymers which will provide efficient catalysis and high flux (mass transported through the membrane in a given time), limited plugging of pores and pressure drop between feed and product streams.

For example, enzymes have been attached to a porous mineral support having a crosslinked polymer covering its surface for separating racemic acetylamino acids in a column. The separation is accomplished by contacting the attached enzyme with an aqueous solution of the racemic mixture. The product stream is the same as the feed stream in the packed-bed column, except for the conversion of one enantiomorph into the other.

Similar materials are described in U.S. Patent 4,251,631 wherein enzymes are attached to crosslinked polymeric membranes and an aqueous feed stream containing substrate is contacted with it under pressure. The feed stream can contain up to 50% of a water-miscible solvent, for example ethanol. Conversion of up to 87% (see Example 1, Col. 9) is allegedly obtained.

It is known to use a batch enzymatic process for stereospecific resolution of a d,L-phenylglycine derivative using a solution composed of water and a water-miscible solvent, such as dioxane, ethanol or acetonitrile. High conversion is possible only after performing several solvent extraction steps to remove the product from the solution. Such a cumbersome process is not adaptable to large-scale commercial production. A similar batch process has been tried using a mixture of water and a water-immiscible solvent containing the appropriate enzyme.

Higher yields (up to 90% optical purity) are apparently obtained with a continuous process described by Matson in a presentation made at Biotech 85 in Geneva, Switzerland in May of 1985. The Matson process utilizes a bioreactor composed of an immobilized liquid membrane laminated to a cellulose membrane to which enzymes are attached. An aqueous feed stream containing the appropriate substrate is moved continuously across the immobilized liquid membrane of the bioreactor.

However, this process and enzymatic bioreactor has a number of disadvantages despite its relatively high resolution of optical isomers. The immobilized liquid membrane containing a porous polymer and an immobilized organic solvent is unstable upon prolonged operation with an aqueous feed stream. The organic solvent eventually leaches out of the membrane. The bioreactor is hard to manufacture and while the optical purity of the isomer is higher than prior processes, it is marginal for commercialization. It would be desirable to have a bioreactor and process which would provide high enough yields from the enzymatic reaction to be practical for commercialization and which is useful for a long period of time.

The problems noted above with regard to known processes are avoided with a method for effecting enzymatic reactions with a microporous enzymatic reactor comprising a porous support having thereon a polymeric membrane having an enzyme attached thereto, the method comprising:

causing a feed stream containing a substrate for the enzyme dissolved in a first solvent to flow rapidly and continuously over the surface of the membrane, and

collecting the product of enzymatic reaction occurring in the membrane in a product stream which is separated from the feed stream by the enzymatic membrane,

the method characterized wherein the membrane is crosslinked and contains solubilizing groups and the product stream comprises a second solvent which is immiscible with the first solvent.

This invention also provides a microporous enzymatic reactor comprising a porous support having thereon a polymeric membrane having an enzyme attached thereto,

the membrane characterized wherein it is prepared from a polymer comprised of recurring units derived from acrylamide or methacrylamide monomers containing active methylene groups, and recurring units derived from solubilizing ethylenically unsaturated monomers, and crosslinked with a crosslinking agent capable of reacting with the active methylene groups.

Further, the present invention provides a method for the preparation of the enzymatic reactor described above comprising:

coating, on a porous support, a solution buffered to from 4.5 to 5.5 and containing a polymer comprising recurring units derived from acrylamide or methacrylamide monomers containing active methylene groups, and recurring units derived from solubilizing ethylenically unsaturated monomers, an enzyme and a crosslinking agent, and

drying the resulting coating to provide a crosslinked polymeric film.

The present invention provides a means for obtaining high yields from an enzymatic reaction in a continuous production operation. In particular, it provides improved separation of racemic mixtures of enantiomorphs using enzymatic reactions of one enantiomorph of the mixture. The bioreactor is easily fabricated and has a long useful life.

These advantages are possible by use of a crosslinked polymeric membrane which also has hydrophilicity provided by solubilizing groups attached to the polymer backbone. In the method of this invention, the substrate for the enzyme in the bioreactor is dissolved in a feed stream composed essentially of a first solvent which is immiscible with the solvent of the product stream containing the resulting enzymatic product.

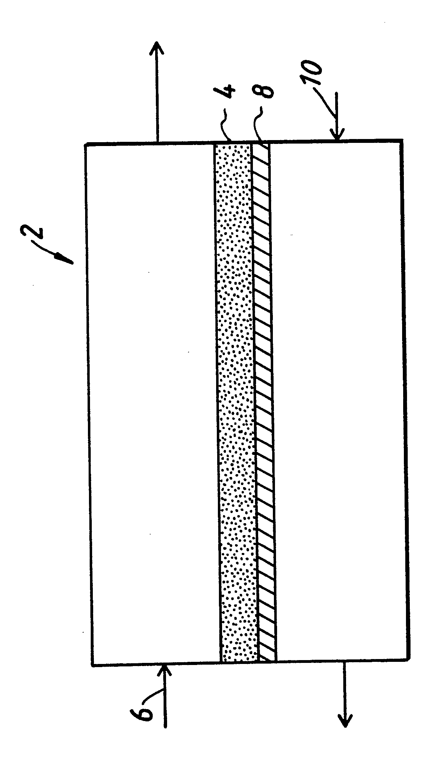

The figure is a schematic illustration of the enzymatic reactor of the present invention.

As stated above, the present invention provides a method for effecting enzymatic reactions using the microporous enzymatic reactor described herein. A substrate for the enzyme is dissolved in a feed stream comprising a first solvent in which the substrate is soluble, but in which the enzymatic product is insoluble. Any enzyme suitable for causing enzymatic reactions can be used in this invention. Thus, an unrestricted number of possibilities for the performance of enzymatic reactions can be considered in the scope of this invention. The product from the enzymatic reaction is taken away in a product stream containing a second solvent. The first and second solvents are immiscible. For example, the first solvent can be water, and the second solvent can be a water-immiscible organic solvent. Preferably, the first solvent is a water-immiscible organic solvent, and the second solvent is water. Alternatively, the two solvents can be immiscible organic solvents. The first and second solvents can be individual mixtures of miscible solvents as long as the mixtures are immiscible. The feed stream contains substantially none of the second solvent, and the product stream contains substantially none of the first solvent.

In a preferred embodiment of this invention, the method is used to resolve racemic mixtures of amino acids or amino acid derivatives, such as esters of amino acids. Therefore, a racemic mixture is contacted with the enzymatic reactor during which one enantiomorph penetrates the membrane of the reactor, is converted by enzymatic action to a product which is soluble in the product stream. The second enantiomorph in the feed stream is not converted by the enzyme and remains in the feed stream. The resulting product can then be isolated from the product stream and used as is or used as an intermediate to prepare another material using known techniques. One can choose the derivatives for the feed stream in order to fix the relative solubilities of reactants in the feed and product streams. This will enhance reaction rates and reduce product stream contamination.

Enzymes useful in the practice of this invention can be of animal, microbial or plant origin. For example, hydrolases (such as chymotrypsin, trypsin, amylases, amino acylase and carboxypeptidase), oxidases (such as glucose oxidase, catalase, alcohol dehydrogenase and peroxidase), amidases (such as urease and asparaginase), alkaline phosphatase, acid phosphatase can be used as well as others known in the art. The enzyme can be attached to the membrane in any suitable way, including for example, entrapment, covalent reaction with reactive groups in the membrane, adsorption, electrostatic interaction and other techniques known in the art.

The enzymatic reactor of this invention is prepared from a porous support having at least 10% porosity, and preferably at least 30% porosity, such as a porous polypropylene (available as CELGARD 2500 from Celanese), porous TEFLON (available as GORE-TEX from W. L. Gore & Associates, Inc.), porous poly(vinylidene fluoride) (available as DURAPORE from Millipore Co.), porous poly(vinyl chloride) (available as PVC-5 from Millipore Co.), and others known in the art. Porous polypropylene is preferred in practising this invention.

A membrane solution comprising a suitable polymer and a suitable enzyme is coated onto the porous support to form a crosslinked polymeric membrane. This solution is carefully buffered to a pH of from 4.5 to 5.5 in order to prevent premature crosslinking and to keep swelling from 2 to 10 times the original coating thickness. If swelling is less than 2 times, the diffusion rate is too slow and the overall flux is low. If swelling is more than 10 times, the enzyme can readily leach out of the membrane, but some swelling is desirable to promote diffusion of substrate therein. Immediately after coating, the coated polymer composition is crosslinked in a suitable manner and dried. The polymer composition can contain one or more suitable crosslinking agents which react with the active methylene groups of the polymer to provide a crosslinked matrix when the coating solvent is removed. Any crosslinking agent can be used as long as it does not adversely affect the enzyme in the membrane of the reactor. Different crosslinking agents may affect various enzymes differently. Representative crosslinking agents include formaldehyde, glutaraldehyde, succinaldehyde, mucochloric acid, bis(vinylsulfonylmethyl) ether, bis-(vinylsulfonyl)methane and others known to one skilled in the art. Other suitable means of crosslinking include the use of epoxides, aziridines, α-diketones, blocked dialdehydes, active halogens, carbodiimides, isoxazolium salts, or other compounds known to be useful as crosslinking agents for active-methylene group-containing polymers. Crosslinking can occur during or after drying, or both.

The polymers useful in the practice of this invention include any crosslinkable synthetic or natural resin which contains sufficient solubilizing groups (for example, sulfonate, carboxyl, sulfate, phosphonate, and others known in the art) to provide desired hydrophilicity. The resulting polymer has a sufficient balance of hydrophilicity and crosslinking in order to obtain the desired swelling characteristics described above. Preferably, the solubilizing groups are sulfonate or carboxylate groups, or a mixture of both. In a preferred embodiment, the polymers useful in the practice of this invention are comprised of: (a) from 2 to 75 weight percent of recurring units derived from one or more acrylamide or methacrylamide monomers containing active methylene groups, (b) from 25 to 98 weight percent of recurring units derived from one or more solubilizing ethylenically unsaturated monomers, and (c) optionally, from 0 to 50 weight percent of recurring units derived from one or more other ethylenically unsaturated polymerizable monomers. In preferred embodiments, the polymers contain from 25 to 75 weight percent of (a), from 25 to 75 weight percent of (b) and from 0 to 25 weight percent of (c).

Useful monomers from which (a) can be derived include
N-(3-acetoacetamidopropyl)methacrylamide,
N-(3-acetoacetamidopropyl)acrylamide,
N-(2-acetoacetoxyethyl)acrylamide,
N-(2-acetoacetoxyethyl)methacrylamide,
N-(2-acetoacetamidoethyl)methacrylamide,
N-(2-acetoacetamidoethyl)acrylamide and others known to one skilled in the art, as evidenced by the disclosure in U.S. Patent 4,247,673.

Useful monomers from which (b) can be derived include sodium 2-acrylamido-2-methyl-propanesulfonate, sodium 3-acryl-oyloxypropanesulfonate, sodium 3-acryloyloxy-1-methylpropanesulfonate, sodium 4-acryloyloxybutanesulfonate, sodium p-styrene sulfonate, sodium methacrylate, sodium acrylate, sodium 2-acryloyloxyethylsulfate and others known to one skilled in the art as evidenced by the disclosure of U.S. Patents 3,506,707, 3,547,899, 2,923,734, 3,024,221, 3,411,911 and 3,265,654.

Optional units (c) can be derived from 2-hydroxyethyl acrylate, 2-hydroxyethyl methacrylate, acrylamide, methacrylamide and other monomers known to one skilled in the art that will not adversely affect the membranes hydrophilicity and crosslinking properties.

Representative polymers useful in this invention include poly[sodium 2-acrylamido-2-methylpropanesulfonate-co-N-(3-acetoacetamidopropyl)methacrylamide], poly[N-(3-acetoacetamidopropyl)acrylamide-co-sodium 3-acryloyloxypropanesulfonate], poly[N-(2-acetoacetoxyethyl)acrylamide-co-sodium 3-acryloyloxy-1-methylpropanesulfonate], poly[N-(2-acetoacetamidoethyl)acrylamide-co-sodium 4-acryloyloxybutanesulfonate], and poly[N-(3-acetoacetamidopropyl)methylacrylamide-co-sodium 2-acryloyloxyethylsulfate]. The first polymer is preferred.

Water-immiscible solvents useful in the practice of this invention include toluene, n-decanol, o-dichlorobenzene, o -xylene, hexadecane, iso-octane, cyclohexane and others known to one skilled in the art.

The concentration of substrate in the feed stream can be varied but is generally at least 0.01 molar, and more particularly from 0.1 to 0.5 molar. The feed and product streams are contacted with the membrane of the enzymatic reactor at a sufficient velocity such that concentration polarization is minimal. For example, the linear velocity at the membrane surface can be at least 0.1 cm/sec, and preferably greater that 1 cm/sec., for a 0.4 mm spacing. The feed stream can be recirculated or subjected to treatment to remove byproducts or inhibitors between passes, if desired. The enzymatic reaction is generally carried out at a temperature of at least 10°C, and preferably at higher temperatures to increase the membrane flux.

The pH of an aqueous feed or product stream can be varied widely depending upon the enzymes used in the reactions. For one embodiment using α-chymotrypsin, the aqueous stream is generally buffered to a pH of from 7 to 8.

The enzymatic reactor can be configured in any desirable configuration to provide sufficient surface to feed stream volume for the desired reaction. For example, the reactor can be formed as hollow fibers, tubes, or flat, folded or corrugated sheets.

The figure illustrates schematically the enzymatic membrane reactor 2 of this invention. A porous support 4 of the reactor 2 is contacted with a feed stream 6 containing one solvent and a substrate for the enzyme. The substrate passes through the support 4 and into polymeric membrane 8 where enzymatic reaction occurs. A product stream 10 carries the resulting enzymatic product away from the reactor 2. Stream 10 comprises a solvent which is immiscible with the solvent in the feed stream 6. The figure shows the two streams in a cross-current mode. However, a co-current mode is also useful.

In a preferred embodiment of this invention, the enzymatic reactor is used to separate racemic mixtures of enantiomorphs, such as a racemic mixture of N-acetyl-d,L-phenylalanine alkyl esters (for example, methyl, ethyl, n -propyl or isopropyl esters). The L enantiomorph is hydrolyzed with a hydrolyase such as α-chymotrypsin. Example 2 below illustrates this embodiment in detail.

The following examples illustrate the practice of this invention.

Example 1: Preparation of Enzymatic Reactor

An enzymatic reactor of this invention was prepared in the following manner.

A solution of α-chymotrypsin (5% in phosphate buffer) was mixed with an equal volume of poly-[sodium-2-acrylamido-2-methlypropanesulfonate-co-N-(3-acetoacetamidopropyl)methacrylamide] (weight ratio 25:75) (5% in phosphate buffer). The final pH of the solution was about 5.3. ZONYL FSN fluorinated surfactant (from DuPont Co., Wilmington, Delaware) was added to the solution to give a final concentration of 0.05%. Glutaraldehyde solution (10% concentration) was then added to provide a 2% (dry weight) final crosslinking agent concentration in the final coating.

A porous polypropylene film (CELGARD 2500 available from Celanese) was used as the reactor support. It has a porosity of 45% and a sheet thickness of about 0.0025 mm. After the edges of the support were taped to a glass plate, the solution with the polymer and enzyme described above was coated onto it at a wet thickness of about 0.13 mm and allowed to dry. Crosslinking occurred during drying. The coating thickness was not critically controlled, and the support having the crosslinked membrane thereon was kept in a plastic bag until its use.

Example 2: Enzymatic Reaction Using Enzymatic Reactor

The enzymatic reactor described in Example 1 was used to separate racemic enantiomorphs of an ester of N-acetyl-d,L-phenylalanine in the following manner.

The enzymatic reactor of Example 1 was placed between two compartments of a stainless steel reactor vessel. Meshed nylon screen was used to define the depth of each compartment so as to achieve high linear flow velocity on both sides of the membrane. The organic feed stream and aqueous product stream were circulated in cross flows through the individual compartments. A linear velocity greater than 1 cm/sec. (for a 0.4 mm spacing) of both streams was maintained at the respective surfaces.

The feed stream contained N-acetyl d,L-phenylalanine methyl ester (35 mmolar) in toluene (240 ml). The product stream contained calcium chloride (50 mmolar) in tris(hydroxymethyl)-aminomethane buffer (82 ml of 40 mmolar, pH 7.8).

The production of N-acetyl-L-phenylalanine was accomplished at an initial flux of 1.3 kg/m$^2$/day. The same reactor was used in similar tests four months later, and the initial flux was determined to be 1.1 kg/m$^2$/day with the same feed stream at 18 mmolar N-acetyl-d,L-phenylalanine methyl ester. The concentration of product in the product stream was greater than 462 mmolar, and the equilibrium concentration of the unreacted d-ester was 7 mmolar in the feed stream. When the d-ester was removed by extraction with toluene, the optical purity of N-acetyl L-phenylalanine was determined to be about 99.8%.

Example 3: Separation of Racemic Mixture

Five months after the first test described in Example 2, the enzymatic reactor described therein was used to separate a mixture of N-acetyl-d,L-phenylalanine ethyl ester (100 mmolar) according to the procedure described in that example. The initial flux was determined to be 1.7 kg/m$^2$/day, and the L-enantiomorph was concentrated to 600 mmolar with an equilibrium concentration of 8 mmolar d-ester. The optical purity of the L-enantiomorph was greater than 99.9% after extraction with toluene.

Example 2 was repeated six months later with a feed stream of 35 mmolar N-acetyl-d,L-phenylalanine methyl ester and an initial flux of 1.2 kg/m$^2$/day was measured.

'CELGARD 2500', 'TEFLON', 'GORE-TEX', 'DURAPORE', 'PVC-5' and 'ZONYL FSN', referred to above, are trade marks.

## Claims

1. A method for effecting enzymatic reactions with a microporous enzymatic reactor comprising a porous support having thereon a polymeric membrane having an enzyme attached thereto, the method comprising:

causing a feed stream containing a substrate for the enzyme dissolved in a first solvent to flow rapidly and continuously over the surface of the membrane, and

collecting the product of enzymatic reaction occurring in the membrane in a product stream which is separated from the feed stream by the enzymatic membrane,

the method characterized wherein the membrane is crosslinked and contains solubilizing groups and the product stream comprises a second solvent which is immiscible with the first solvent.

2. The method as claimed in claim 1 wherein the first solvent is water-immiscible organic solvent, and the second solvent is water.

3. The method as claimed in claim 1 or 2 wherein the concentration of the substrate in the feed stream is at least 0.01 molar.

4. The method as claimed in any one of claims 1 to 3 for preparing an optically active amino acid from a racemic mixture of the amino acid with the membrane having an enzyme attached thereto which is capable of stereospecifically reacting with only one enantiomer of the mixture.

5. The method as claimed in claim 4 for preparing L-phenylalanine.

6. A microporous enzymatic reactor comprising a porous support having thereon a polymeric membrane having an enzyme attached thereto,

the membrane characterized wherein it is prepared from a polymer comprised of recurring units derived from acrylamide or methacrylamide monomers containing active methylene groups, and recurring units derived from solubilizing ethylenically unsaturated monomers, and crosslinked with a crosslinking agent capable of reacting with the active methylene groups.

7. The reactor as claimed in claim 6 wherein the membrane polymer is prepared from acrylamide or methacrylamide monomers selected from the group:

N-(3-acetoacetamidopropyl)methacrylamide,
N-(3-acetoacetamidopropyl)acrylamide,
N-(2-acetoacetoxyethyl)acrylamide,
N-(2-acetoacetoxyethyl)methacrylamide,
N-(2-acetoacetamidoethyl)methacrylamide, and
N-(2-acetoacetamidoethyl)acrylamide,

and the solubilizing monomers are selected from the group:

sodium 2-acrylamido-2-methylpropanesulfonate, sodium 3-acryloyloxypropanesulfonate, sodium 3-acryloyloxy-1-methylpropanesulfonate, sodium 4-acryloyloxybutanesulfonate, sodium p-styrenesulfonate, sodium methacrylate, sodium acrylate, and sodium 2-acryloyloxyethylsulfate.

8. A method for the preparation of an enzymatic reactor comprising:

coating, on a porous support, a solution buffered to from 4.5 to 5.5 and containing a polymer comprising recurring units derived from acrylamide or methacrylamide monomers containing active methylene groups, and recurring units derived from solubilizing ethylenically unsaturated monomers, an enzyme and a crosslinking agent, and

drying the resulting coating to provide a crosslinked polymeric membrane.

9. The invention as claimed in any one of claims 1 to 8 wherein the enzyme is α-chymotrypsin.

10. The invention as claimed in any one of claims 1 to 9 wherein the membrane is composed of poly[sodium 2-acrylamido-2-methylpropanesulfonate-co-N-(3-acetoacetamidopropyl)methacrylamide].